# FASCICULE DE BREVET EUROPEEN

(11) **EP 1 675 535 B1**
(45) Date de publication et mention de la délivrance du brevet: **02.05.2007**
(21) Numéro de dépôt: 04787457.3
(22) Date de dépôt: 27.09.2004
(51) Int. Cl.: A61F 2/44

(54) **IMPLANT INTERVERTEBRAL**
BANDSCHEIBENIMPLANTAT
INTERVERTEBRAL IMPLANT

(30) Priorité: 02.10.2003 FR 0311534
(43) Date de publication de la demande: 05.07.2006
(73) Titulaire: Fixano, 01000 Bourg En Bresse (FR)
(72) Inventeur: SGIER, Frédéric, CH-60006 Luzern (CH); MARTIN, Jean-Jacques, F-01000 Bourg-en-Bresse (FR)
(74) Mandataire: Schlich, George William
(86) Numéro de dépôt international: PCT/FR2004/002434
(87) Numéro de publication internationale: WO 2005/032432

(56) Documents cités:
- WO-A-03/026522
- WO-A-03/077806
- FR-A- 2 812 806
- US-A1- 2002 077 702

## Description

La présente invention concerne un implant intervertébral.

Il est bien connu d'insérer un implant rigide, creux intérieurement, entre les corps de deux vertèbres. Un tel implant a pour fonctions de rétablir la distance et la position anatomique de ces vertèbres en cas de disque affaissé, et d'opérer une immobilisation relative de ces vertèbres par croissance des cellules osseuses au travers de l'implant.

L'immobilisation complète des vertèbres l'une par rapport à l'autre a pour inconvénients de réduire la mobilité du patient et de générer des sur-sollicitations sur les articulations sus-jacente et sous-jacente.

Il est par ailleurs connu par le document US 2002/077702 A1 de prévoir un implant intervertébral qui présente deux parois juxtaposées ayant une forme en "U" et reliées l'une à l'autre, au niveau d'un de leurs bords, par une paroi intermédiaire présentant un léger degré de souplesse élastique, cette paroi intermédiaire étant déformable de manière à permettre une mobilité desdites parois juxtaposées l'une par rapport à l'autre.

Ce document décrit un implant pouvant être implanté soit seul soit avec un autre implant identique, et pouvant être mis en place près du bord du plateau vertébral d'une vertèbre. Les branches supérieures et inférieures de cet implant sont séparées deux à deux par des fentes de largeurs limitées.

Cet implant ne permet pas de restaurer l'espace anatomique entre deux vertèbres ni de restituer un jeu fonctionnel approprié de ces vertèbres.

La présente invention vise à remédier à ces inconvénients essentiels.

L'implant qu'elle concerne présente, comme cela est connu, deux parois juxtaposées ayant une forme en "U" et reliées l'une à l'autre, a u niveau d'un de leurs bords, par une paroi intermédiaire présentant un léger degré de souplesse élastique, cette paroi intermédiaire étant déformable de manière à permettre une mobilité desdites parois juxtaposées l'une par rapport à l'autre.

Selon l'invention, l'implant est dimensionné de telle sorte que les branches latérales desdites parois juxtaposées peuvent être engagées, par voie postérieure, de part et d'autre de la moelle épinière et peuvent venir s'insérer entre les plateaux vertébraux des deux vertèbres traitées, dans les zones latérales de ces plateaux.

Lorsque l'implant est mis en place, les branches latérales desdites parois juxtaposées portent contre les plateaux vertébraux des vertèbres traitées, dont elles assurent le rétablissement de la distance anatomique et le repositionnement. Grâce à la déformabilité de ladite paroi intermédiaire, ces parois juxtaposées peuvent jouer l'une par rapport à l'autre pour permettre de conserver la mobilité des vertèbres, évitant ainsi une limitation des mouvements du patient et l'exercice de sur-sollicitations sur les disques intervertébraux sus-jacent et sous-jacent.

La paroi intermédiaire peut être conformée pour permettre une mobilité desdites parois juxtaposées uniquement dans un ou deux plans, c'est-à-dire, après implantation, le plan sagittal, le plan frontal et/ou le plan perpendiculaire à l'axe du rachis. De préférence, toutefois, cette paroi intermédiaire est déformable de manière à permettre une mobilité desdites parois juxtaposées dans trois plans, c'est-à-dire, après implantation, le plan sagittal, le plan frontal et le plan perpendiculaire à l'axe du rachis.

Les branches latérales desdites parois juxtaposées sont avantageusement dimensionnées de telle sorte qu'après mise en place, par voie postérieure, de l'implant entre les plateaux vertébraux des deux vertèbres traitées, ladite paroi intermédiaire se trouve au niveau, ou légèrement en arrière, de la position qu'avaient les facettes des vertèbres traitées avant mise en place de l'implant, cette mise en place impliquant la résection de ces facettes.

Ce positionnement de la paroi intermédiaire s'avère le plus approprié à l'obtention d'une mobilité optimale des vertèbres.

La paroi intermédiaire peut notamment présenter une forme arrondie vue de côté, c'est-à-dire dans le plan sagittal après implantation.

L'implant a ainsi également une forme sensiblement en "U" vu de côté.

Cette paroi intermédiaire peut en outre présenter une forme arrondie vue dans un plan parallèle auxdites parois juxtaposées, ce qui favorise sa déformabilité dans ce plan. Cette déformabilité dans ce plan permet, après implantation, une mobilité des vertèbres en rotation l'une par rapport à l'autre selon l'axe du rachis.

Au moins une des parois juxtaposées peut présenter un degré de déformabilité élastique pour permettre, conjointement à la déformabilité de ladite paroi intermédiaire, une mobilité étendue de ces parois juxtaposées l'une par rapport à l'autre.

L'implant est avantageusement réalisé en une pièce de matériau, de préférence en titane ou alliage incluant du titane.

Les parois juxtaposées peuvent présenter des aspérités de prise d'appui contre les plateaux vertébraux. Ces aspérités assurent l'immobilisation de l'implant en position et préviennent le risque d'expulsion de l'implant sous l'effet des mouvements répétés des vertèbres.

Ces aspérités peuvent notamment être sous forme de nervures faisant saillie des faces externes desdites parois juxtaposées, et disposées transversalement auxdites branches latérales. Ces nervures peuvent notamment avoir une section transversale triangulaire définissant une arête supérieure vive.

Avantageusement, l'implant présente, dans son ensemble, une forme "mousse", c'est-à-dire dépourvue d'angles vifs susceptibles d'être blessants pour des tissus environnants. Les bords latéraux externes desdites parois juxtaposées peuvent ainsi présenter des formes convexes et l'échancrure centrale délimitée par ces parois juxtaposées peut présenter un fond arrondi.

L'ensemble confère de cette manière à l'implant une forme arrondie, légèrement "en fer à cheval".

Les extrémités libres desdites branches latérales peuvent également présenter une forme arrondie.

L'invention sera bien comprise, et d'autres caractéristiques et avantages de celle-ci apparaîtront, en référence au dessin schématique annexé, représentant, à titre d'exemple non limitatif, une forme de réalisation préférée de l'implant qu'elle concerne.
La figure 1 en est une vue en perspective ;
la figure 2 en est une vue en perspective, sous un autre angle ;
la figure 3 en est une vue de côté ;
la figure 4 en est une vue en plan ;
la figure 5 en est une vue en bout, et
les figures 6 à 8 en sont des vues en perspective, après implantation entre deux vertèbres, selon trois angles différents.

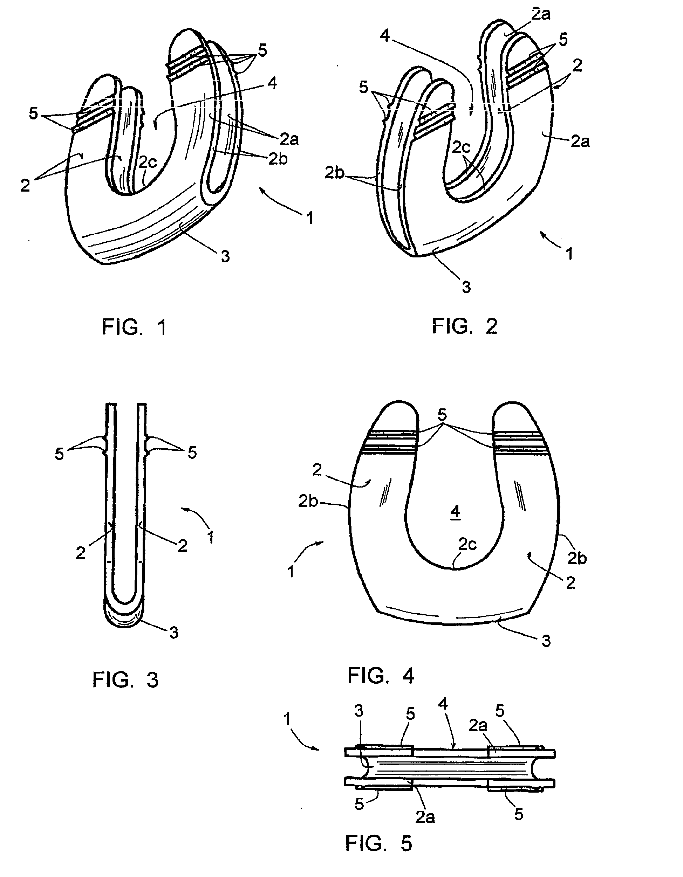

Les figures 1 à 5 représentent un implant intervertébral 1 comprenant deux parois juxtaposées 2 et une paroi intermédiaire 3. L'implant 1 est réalisé en une pièce d'alliage de titane-vanadium.

Les deux parois juxtaposées 2 ont, vues en plan (cf. figure 4), une forme en "U" et sont reliées l'une à l'autre, au niveau d'un de leurs bords, par la paroi 3.

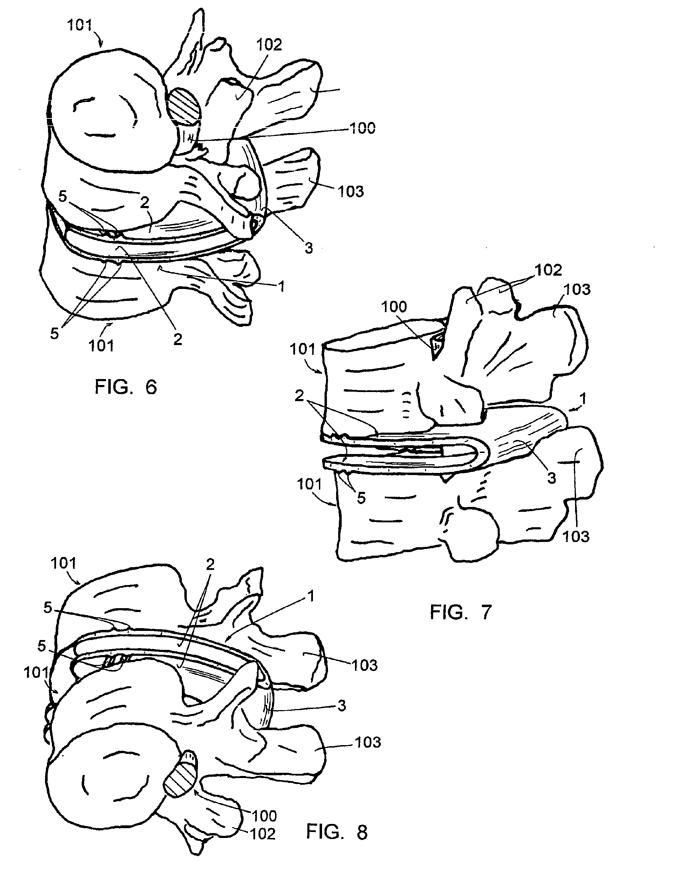

Ainsi que cela est visible sur les figures 6 à 8, l'implant 1 est dimensionné de telle sorte que les échancrures 4 que délimitent les parois 2 permettent un engagement de cet implant 1, par voie postérieure, de part et d'autre de la moelle épinière 100, et un engagement des branches latérales 2a formées par ces échancrures 4 entre les plateaux vertébraux des deux vertèbres 101 traitées, dans les zones latérales de ces plateaux.

L'implant 1 est en outre dimensionné de telle sorte qu'après mise en place, ladite paroi intermédiaire 3 se trouve au niveau, ou légèrement en arrière, de la position qu'occupaient les facettes 102 des vertèbres 101 avant mise en place de l'implant 1, cette mise en place impliquant la résection de ces facettes 102.

La paroi intermédiaire 3 présente, comme cela est visible sur la figure 3, une forme arrondie vue de côté, c'est-à-dire dans le plan sagittal après implantation. Elle présente également, comme cela est visible sur la figure 4, une forme arrondie vue dans un plan parallèle aux parois juxtaposées 2.

Les parois juxtaposées 2 présentent en outre quatre paires de nervures 5 faisant saillie de leurs faces externes, disposées transversalement auxdites branches latérales 2a. Ces nervures 5 ont une section transversale triangulaire définissant une arête supérieure vive.

En référence aux figures 1, 2 et 4, il apparaît que l'implant 1 présente, dans son ensemble, une forme "mousse", c'est-à-dire dépourvue d'angles vifs susceptibles d'être blessants pour des tissus environnants. Les bords latéraux externes 2b desdites parois juxtaposées 2 ont des formes convexes, les échancrures 4 présentent un fond arrondi 2c, et les extrémités libres des branches latérales 2a ont une forme arrondie. L'ensemble confère à l'implant 1 une forme légèrement "en fer à cheval".

En pratique, comme cela apparaît en référence aux figures 6 à 8, les facettes 102 et les deux apophyses épineuses 103 des vertèbres 101 sont réséquées pour aménager un espace permettant le passage de l'implant 1. Cet implant 1 peut ainsi être engagé dans cet espace, par voie postérieure, jusqu'à ce que les branches latérales 2a des parois 2 soient insérées entre les plateaux vertébraux des vertèbres 101. Cet engagement est rendu possible par les échancrures 4, lesquelles aménagent des dégagements suffisants pour éliminer tout risque de contact de l'implant 1 avec la moelle épinière 100.

En position d'implantation, la paroi 3 est située au niveau de la position qu'avaient, avant résection, les facettes 102, ou légèrement en arrière de celle-ci, et les nervures 5 prennent appui dans les plateaux pour éliminer tout risque de recul ou d'expulsion de l'implant 1 sous l'effet des mouvements répétés que les vertèbres 101 exercent sur lui.

Les parois juxtaposées 2 et la paroi intermédiaire 3 ont, de par leur matériau constitutif et de par la forme de la paroi 3, un léger degré de souplesse élastique, tel qu'une mobilité desdites parois juxtaposées 2 est possible dans trois plans, c'est-à-dire, après implantation, dans le plan sagittal (celui de la figure 3), dans le plan frontal (celui de la figure 5), et dans le plan perpendiculaire à l'axe du rachis (celui de la figure 4) pour une mobilité des vertèbres en rotation l'une par rapport à l'autre selon l'axe du rachis.

Comme cela apparaît de ce qui précède, l'invention fournit un implant intervertébral 1 présentant l'avantage déterminant de pouvoir assurer le rétablissement de la distance anatomique des vertèbres 101 et le repositionnement anatomique de ces vertèbres, tout en permettant à ces vertèbres, grâce à la déformabilité de ladite paroi intermédiaire 3 et des parois juxtaposées 2, de jouer l'une par rapport à l'autre. La mobilité des vertèbres, ainsi conservée, évite une limitation des mouvements du patient et évite l'exercice de sur-sollicitations sur les disques intervertébraux sus-jacent et sous-jacent.

Il va de soi que l'invention n'est pas limitée à la forme de réalisation décrite ci-dessus à titre d'exemple mais qu'elle s'étend à toutes les formes de réalisations couvertes par les revendications ci-annexées.

## Revendications

1. - Implant intervertébral (1), présentant deux parois juxtaposées (2) ayant une forme en "U" et reliées l'une à l'autre, au niveau d'un de leurs bords, par une paroi intermédiaire (3) présentant un léger degré de souplesse élastique, cette paroi intermédiaire (3) étant déformable de manière à permettre une mobilité desdites parois juxtaposées (2) l'une par rapport à l'autre, **caractérisé en ce qu'**il est dimensionné de telle sorte que les branches latérales (2a) desdites parois juxtaposées (2) peuvent être engagées, par voie postérieure, de part et d'autre de la moelle épinière (100) et peuvent venir s'insérer entre les plateaux vertébraux des deux vertèbres (101) traitées, dans les zones latérales de ces plateaux.

2. - Implant intervertébral (1) selon la revendication 1, **caractérisé en ce que** la paroi intermédiaire (3) est déformable de manière à permettre une mobilité desdites parois juxtaposées (2) dans trois plans, c'est-à-dire, après implantation, le plan sagittal, le plan frontal et le plan perpendiculaire à l'axe du rachis.

3. - Implant intervertébral (1) selon la revendication 1 ou la revendication 2, **caractérisé en ce que** les branches latérales (2a) desdites parois juxtaposées (2) sont dimensionnées de telle sorte qu'après la mise en place par voie postérieure, de l'implant (1) entre les plateaux vertébraux des deux vertèbres (101) traitées, ladite paroi intermédiaire (3) se trouve au niveau, ou légèrement en arrière, de la position qu'avaient les facettes (102) des vertèbres traitées (101) avant mise en place de l'implant (1), cette mise en place impliquant la résection de ces facettes (102).

4. - Implant intervertébral (1) selon l'une des revendications 1 à 3, **caractérisé en ce que** la paroi intermédiaire (3) présente une forme arrondie vue de côté, c'est-à-dire dans le plan sagittal après mise en place.

5. - Implant intervertébral (1) selon l'une des revendications 1 à 4, **caractérisé en ce que** la paroi intermédiaire (3) présente une forme arrondie vue dans un plan parallèle auxdites parois juxtaposées (2).

6. - Implant intervertébral (1) selon l'une des revendications 1 à 5, **caractérisé en ce qu'**au moins une des parois juxtaposées (2) présente un degré de déformabilité élastique.

7. - Implant intervertébral (1) selon l'une des revendications 1 à 6, **caractérisé en ce qu'**il est réalisé en une pièce de matériau, de préférence en titane ou alliage incluant du titane.

8. - Implant intervertébral (1) selon l'une des revendications 1 à 7, **caractérisé en ce que** les parois juxtaposées (2) présentent des aspérités (5) de prise d'appui contre les plateaux vertébraux.

9. - Implant intervertébral (1) selon l'une des revendications 1 à 8, **caractérisé en ce qu'**il présente, dans son ensemble, une forme dépourvue d'angles vifs susceptibles d'être blessants pour des tissus environnants.

10. - Implant intervertébral (1) selon la revendication 9, **caractérisé en ce que** les bords latéraux externes (2b) desdites parois juxtaposées (2) présentent des formes convexes et **en ce que** l'échancrure (4) délimitée par ces parois juxtaposées (2) présente un fond arrondi.

## Claims

1. Intervertebral implant (1), having two U-shaped juxtaposed walls (2) connected together, in the region of their edges, by an intermediate wall (3) having a slight degree of elastic flexibility, this intermediate wall (3) being deformable so as to allow mobility of said juxtaposed walls (2) in relation to each other, **characterized in that** it is dimensioned so that the lateral branches (2a) of said juxtaposed walls (2) can be engaged, by the posterior route, either side of the spinal chord (100) and can be inserted between the vertebral plates of two treated vertebrae (101), in the lateral zones of these plates.

2. Intervertebral implant (1) according to Claim 1, **characterized in that** the intermediate wall (3) is deformable so as to allow mobility of said juxtaposed walls (2) in three planes, that is to say, after implantation, the sagittal plane, the frontal plane and the plane perpendicular to the axis of the spinal column.

3. Intervertebral implant (1) according to Claim 1 or Claim 2, **characterized in that** the lateral branches (2a) of said juxtaposed walls (2) are dimensioned so that after the implant (1) has been put in place by a posterior route between the vertical plates of the two treated vertebrae (101), said intermediate wall (3) is situated at, or slightly behind, the position that the facets (102) of the treated vertebrae (101) had before the implant (1) was inserted, this insertion involving resection of these facets (102).

4. Intervertebral implant (1) according to one of Claims 1 to 3, **characterized in that** the intermediate wall (3) has a rounded form seen from the side, that is to say in the sagittal plane after insertion.

5. Intervertebral implant (1) according to one of Claims 1 to 4, **characterized in that** the intermediate wall (3) has a rounded shape seen in a plane parallel to said juxtaposed walls (2).

6. Intervertebral implant (1) according to one of claims 1 to 5, **characterized in that** at least one of the juxtaposed walls (2) has a degree of elastic deformability.

7. Intervertebral implant (1) according to one of Claims 1 to 6, **characterized in that** it is made from one piece of material, preferably made of titanium or an alloy including titanium.

8. Intervertebral implant (1) according to one of Claims 1 to 7, **characterized in that** the juxtaposed walls (2) have asperities (5) for bearing against the vertebral plates.

9. Intervertebral implant (1) according to one of Claims 1 to 8, **characterized in that** it has, in its entirety, a shape lacking sharp angles capable of causing injuries to the surrounding tissues.

10. Intervertebral implant (1) according to Claim 9, **characterized in that** the outer lateral edges (2b) of said juxtaposed walls (2) have convex shapes and **in that** the recess (4) delimited by these juxtaposed walls (2) has a rounded bottom.

## Patentansprüche

1. Bandscheibenimplantat (1) mit zwei nebeneinanderliegenden Wänden (2), die eine "U"-Form haben und die an einem ihrer Ränder durch eine Zwischenwand (3) miteinander verbunden sind, die einen leichten Grad an elastischer Biegsamkeit aufweist, wobei diese Zwischenwand (3) deformierbar ist, um eine Beweglichkeit der erwähnten nebeneinanderliegenden Wände (2) gegeneinander zu erlauben, **dadurch gekennzeichnet, dass** es derart bemessen ist, dass die seitlichen Schenkel (2a) der erwähnten nebeneinanderliegenden Wände (2) von der Rückseite her beidseitig des Rückenmarks (100) eingeführt werden können und sich einfügen können zwischen die Wirbelplatten der zwei behandelten Wirbel in den seitlichen Zonen dieser Platten.

2. Bandscheibenimplantat (1) gemäss Anspruch 1, **dadurch gekennzeichnet, dass** die Zwischenwand (3) in der Art beweglich ist, um eine Beweglichkeit der erwähnten nebeneinanderliegenden Wände (2) in drei Ebenen zu ermöglichen, d.h. nach Implantation in der sagittalen Ebene, der frontalen Ebene und der Ebene senkrecht zur Achse der Wirbelsäule

3. Bandscheibenimplantat (1) gemäss Anspruch 1 oder Anspruch 2, **dadurch gekennzeichnet, dass** die seitlichen Schenkel (2a) der genannten nebeneinanderliegenden Wände (2) derart bemessen sind, dass nach dem Einsetzen des Implantats (1) von der Rückseite her zwischen die Wirbelplatten der zwei behandelten Wirbel (101) die besagte Zwischenwand (3) sich auf Höhe, oder geringfügig dahinter, der Position befindet, welche die Facettengelenke (102) der behandelten Wirbel vor dem Einsetzen des Implantats (1) eingenommen hatten, wobei dieses Einsetzen die operative Entfernung dieser Facettengelenke (102) erfordert.

4. Bandscheibenimplantat (1) gemäss einem der Ansprüche 1 bis 3, **dadurch gekennzeichnet, dass** die Zwischenwand (3) von der Seite betrachtet eine abgerundete Form aufweist, d.h. in der sagittalen Ebene nach dem Einsetzen.

5. Bandscheibenimplantat (1) gemäss einem der Ansprüche 1 bis 4, **dadurch gekennzeichnet, dass** die Zwischenwand (3) bei Betrachtung in einer Ebene parallel zu den besagten gegenüberliegenden Wänden (2) eine abgerundete Form aufweist

6. Bandscheibenimplantat (1) gemäss einem der Ansprüche 1 bis 5, **dadurch gekennzeichnet, dass** mindestens eine der nebeneinanderliegenden Wände (2) eine gewisse elastische Deformierbarkeit aufweist.

7. Bandscheibenimplantat (1) gemäss einem der Ansprüche 1 bis 6, **dadurch gekennzeichnet, dass** es aus einem Materialstück, vorzugsweise aus Titan oder aus einer Titan enthaltenden Legierung hergestellt ist.

8. Bandscheibenimplantat (1) gemäss einem der Ansprüche 1 bis 7, **dadurch gekennzeichnet, dass** die nebeneinanderliegenden Wände (2) Unebenheiten (5) zur Verankerung gegenüber den Wirbelplatten aufweisen.

9. Bandscheibenimplantat (1) gemäss einem der Ansprüche 1 bis 8, **dadurch gekennzeichnet, dass** es in seiner Gesamtheit eine Form ohne spitze Ecken aufweist, welche für die umliegenden Gewebe verletzend sein könnten.

10. Bandscheibenimplantat (1) gemäss Anspruch 9, **dadurch gekennzeichnet, dass** die äusseren seitlichen Ränder (2b) der besagten nebeneinanderliegenden Wände (2) konvexe Formen aufweisen und dass der Einschnitt (4) begrenzt durch die beiden nebeneinanderliegenden Wände eine abgerundete Form aufweist.
